# EUROPEAN PATENT APPLICATION

(11) **EP 1 421 901 A2**
(43) Date of publication of application: **26.05.2004**
(21) Application number: 03025123.5
(22) Date of filing: 03.11.2003
(51) Int. Cl.: A61B 5/08

(54) **Arrangement for passive gas sampling**

(30) Priority: 20.11.2002 SE 0203426
(71) Applicant: Maquet Critical Care AB, 171 95 Solna (SE)
(72) Inventor: Castor, Rolf, 129 42 Hägersten (SE); Hallbäck, Magnus, 167 38 Bromma (SE)
(74) Representative: Samzelius, Roger Mikael

(57) **Abstract**

An arrangement (2) for passive gas sampling of a breathing gas in a breathing system is described. The arrangement (2) comprises an element (8) arranged in a tube (4) for the passive diversion of a portion of the flow of breathing gas to a measurement chamber (6).

## Description

The present invention relates to an arrangement for passive gas sampling according to the preamble of Claim 1.

In breathing systems such as ventilators and anaesthetic apparatus the breathing gas is analysed regularly. This may be done directly in the main supply (with a so-called mainstream-analyser) or by diverting a gas sample to a measuring chamber (so-called sidestream-analyser).

The diversion of the gas sample can be done actively by means of a pump or the like or passively, for example by creating a pressure variation between the pressure chamber's inlet and outlet. An example of the latter can be found described in US 6, 450, 968.

An aim of the present invention is to provide an alternative embodiment of an arrangement for passive gas sampling.

This aim is achieved according to the invention by means of the arrangement according to above being adapted as evidenced by the characterising portion of claim 1.

Advantageous refinements and embodiments are set out in the claims dependent on claim 1.

A gas sample may be easily obtained passively using an element that diverts the gas sample from a flow of breathing gas to a measurement chamber. The element can take different forms in order to effectively divert the gas sample and furthermore can be designed to divert a gas sample in one or two flow directions.

The aim is also achieved by means of an arrangement according to the above adapted according to the characterising portion of claim 4.

This solution builds upon the same principle, but instead of an element that diverts the gas sample, here the actual arrangement is designed to, in principle, force the whole gas flow to pass the measurement chamber.

Exemplary embodiments of arrangements according to the present invention shall be described with reference to the figures, of which:
FIG. 1 shows a first embodiment of an arrangement according to the invention;
FIG. 2 shows an element in the arrangement according to the first embodiment;
FIG. 3 shows a second embodiment of a arragement according to the invention; and
FIG. 4 shows a third embodiment of an arrangement according to the invention.

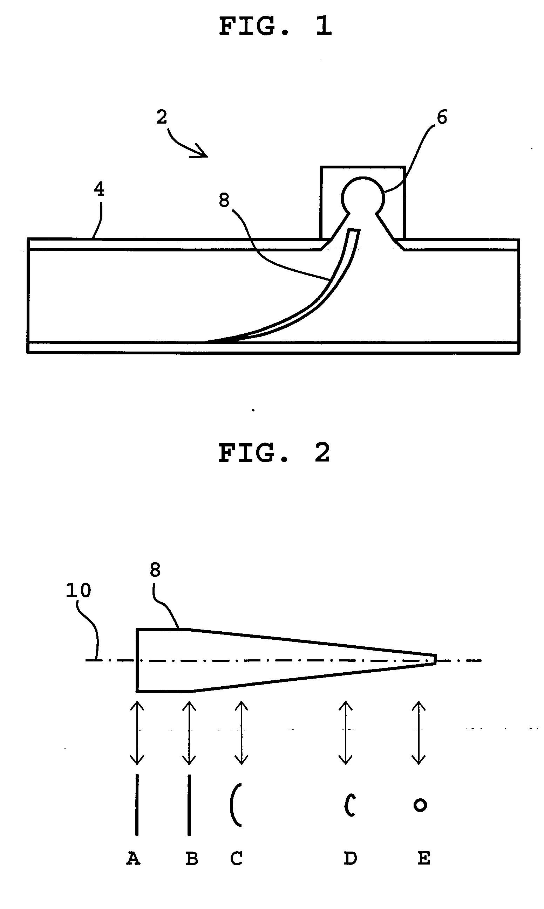

A first embodiment of an arrangement 2 according to the invention is shown in FIG. 1. The arrangement 2 is arranged in a tube 4. A gas sample may be analysed in a measurement chamber 6 according to some known method of analysis, e.g. optical, acoustical or electrochemical.

Gas samples are led into the measurement chamber 6 via an element 8 arranged in the tube 4. The element 8 is curved to lead the gas sample into the measurement chamber 6. As is shown in FIG. 2 the element 8 is also curved around a central axis 10. In the lower portion of FIG. 2 the profile is shown at different sections of the element 8. A first section A shows that the element 8 is straight at its base. Similarly with the second section B. Then the element 8 is successively curved, as is shown in a third section C and a fourth section D. At its tip the element 8 finally presents an essentially round ring, as is shown in a fifth section E.

The successive curving around the centre axis 10 contributes to an optimisation of the flow of the gas sample into the measurement chamber 6.

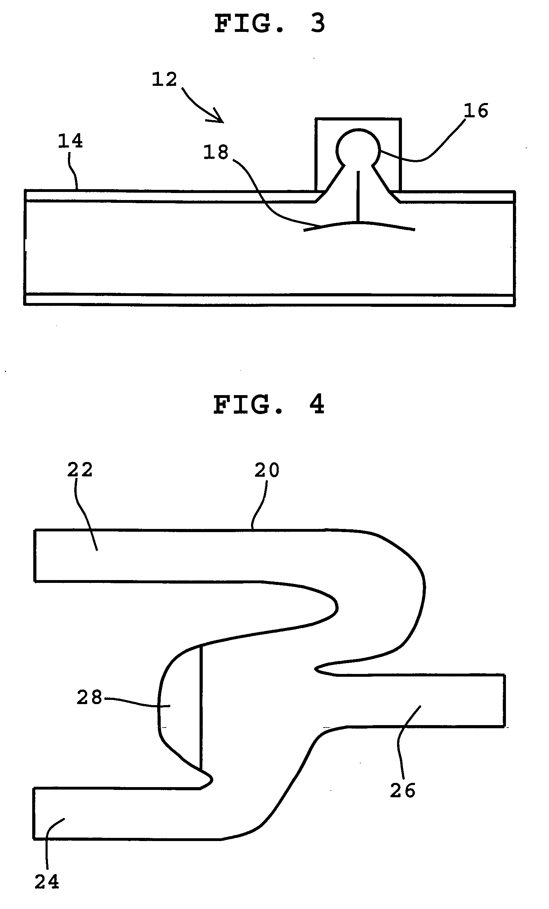

A second embodiment of an arrangement 12 according to the invention is shown in FIG. 3. The arrangement 12 is arranged in a tube 14. A gas sample may be analysed in a measurement chamber 16 according to some known method of analysis. An element 18 is arranged in the tube 14 to lead a gas sample into the measurement chamber 16.

Unlike the first exemplary embodiment, the arrangement 12 according to the second exemplary embodiment is designed to sample gas in both flow directions within the tube 14. This can be done by providing the element 18 with an extension in both the directions. The arrangement 12 is therefore suitable for placement near a patient and to sample gas during both inspiration and expiration.

The gas sample will be led in from one side (from the left side in the figure when the gas flow is from the left to the right and from the right side when the gas flow is from the right to the left) and at the same time the contents of the measurement chamber 16 is forced out through the other side (right respectively left).

A third embodiment of an arrangement 20 according to the invention is shown in FIG. 4.

The arrangement 20 has a first connection 22 for reception of an inspiration flow from a (not shown) breathing arrangement, a second connection 24 for expulsion of an expiration flow and a third connection 26 for expulsion of the inspiration flow and reception of the expiration flow to and respectively from a (not shown) patient. A measurement chamber 28 is formed in the arrangement 20 where a gas sample can be analysed in a known way. The arrangement 20 is designed so that the first connection 22 and the third connection 26 lead respectively the received inspiration flow and expiration flow essentially straight towards the measurement chamber 28.

The measurement chamber 28 is essentially completely open to the flow paths in the arrangement 20, which is why it actually constitutes a cross between a mainstream-analyser and a sidestream-analyser. Gas samples are efficiently swapped during both inspiration and expiration as the breathing gas flow is then directed towards the measurement chamber 28.

Combinations of the described exemplary embodiments can easily be done. For example the design of the element 8 in FIG. 1 can even be used for the element 18 in FIG. 3.

## Claims

1. An arrangement (2; 12) for passive gas sampling of a breathing gas in a breathing system, **characterised in that** the arrangement (2; 12) comprises an element (8; 18) arranged in a tube (4; 14) for the passive diversion of a portion of the flow of breathing gas to a measurement chamber (6; 16).

2. An arrangement according to Claim 1, **characterised in that** the element (8) is formed of an extended, essentially arrowhead-like thin plate having a tip and a base and that is curved in part along the extension and in part about a central axis (10) extending from the tip to the base, such that the plate provides a tubal profile (E) at the tip and a flat profile (A) at the base.

3. An arrangement according to Claim 1, **characterised in that** the element (18) is formed of a deflector having an essentially T-shaped cross-section and arranged with the stem in towards the measurement chamber (16).

4. An arrangement (20) for passive gas sampling of a breathing gas in a breathing system, **characterised in that** the arrangement (20) comprises a first connection (22) for reception of an inspiration flow, a second connection (24) for expulsion of an expiration flow and a third connection 26 for expulsion of the inspiration flow and reception of the expiration flow, and a measurement chamber (28), whereby the arrangement (20) is configured so that the first connection (22) and the third connection (26) lead respectively the received inspiration flow and expiration flow essentially straight towards the measurement chamber (28).
